**Europäisches Patentamt**

(19) **European Patent Office**

**Office européen des brevets**

(11) Publication number: **0 252 033 B1**

# EUROPEAN PATENT SPECIFICATION

(45) Date of publication of the patent specification:
27.12.90

(51) Int. Cl.⁵: **A61K 31/195, A61K 47/10**

(21) Application number: **87830254.6**

(22) Date of filing: **02.07.87**

(54) A "gel" pharmaceutical form containing N-(2.6-Dichloro-M-Tolil)-Anthranilic acid (mechlophenamic acid) for use in therapy by topical application.

(30) Priority: **02.07.86 IT 942986**

(43) Date of publication of application:
**07.01.88 Bulletin 88/1**

(45) Publication of the grant of the patent:
**27.12.90 Bulletin 90/52**

(84) Designated Contracting States:
**AT BE CH DE ES FR GB GR LI LU NL SE**

(56) References cited:
**EP-A- 0 147 476
BE-A- 886 486
GB-A- 1 402 282
GB-A- 2 138 287**

(73) Proprietor: **A. MENARINI INDUSTRIE FARMACEUTICHE RIUNITE S.R.L., Via Sette Santi 3, I-50131 Firenze(IT)**

(72) Inventor: **Bani, Raffaello, Via Val di Chiana N. 56, I-50127 Firenze(IT)**
Inventor: **Ghelardoni, Mario, Via Lambruschini N. 5, I-50134 Firenze(IT)**
Inventor: **Melani, Francesco, Via Poggio Magherini N. 5, I-50014 Fiesole Firenze(IT)**

(74) Representative: **Mannucci, Gianfranco, Dott.-Ing., Ufficio Tecnico Ing. A. Mannucci Via della Scala 4, I-50123 Firenze(IT)**

ACTORUM AG

## Description

The invention refers to a pharmaceutical form containing N-(2.6-dichloro-m-tolyl)-anthranilic acid (meclophenamic acid) as an active compound for topical use, made up of a preparation of the product in a gel form.

GB-A 2 138 287 discloses a non-aqueous clear gel of meclophenamic acid in a co-solvent system of a polyethylene glycol ester, water-soluble lanolin oil, an alcohol and a thickening agent. In this known formulation, the active compound forms a solution in a greasy carrier. Its application on the skin leaves a layer of greasy substances which is unpleasant for the patient and which, moreover, hinders a correct perspiration of the skin.

EP-A 0 147 476 discloses a gel preparation for topical use comprising diclofenac sodium as an active compound in a hydro-alcoholic medium. To the solution of diclofenac sodium and hydro-alcoholic solvent there is added a high percentage of glycol which ensures a good absorption of the active compound by the skin. The presence of glycols in the gel preparation renders the product unpleasant for the patient as these groups of chemical compounds leave a sticky layer on the skin which, again, hinders a correct perspiration.

It was now surprisingly recognized that a suspension of meclophenamic acid in a hydro-alcoholic medium (i.e. not in a co-solvent system formed by oily compounds) allows a very good adsorption of the active compound, even in the absence of glycols. This new pharmaceutical form has excellent local and general tolerability and anti-inflammatory and analgetic activity. The invention allows an efficacious local percutaneous treatment of the algesic, phlogistic, or traumatic conditions of the articulations, tendons, ligaments and muscles.

The pharmaceutical form according to the invention is made sufficiently viscous by means of suitable excipients in order to ensure that it can be smeared onto the injured part. This pharmaceutical form represents a new means for administering the meclophenamic acid for topical usage, and is different from the most common topical forms. It allows a complete adsorption of the active compound and does not leave a layer of greasy or sticky substances which could hinder perspiration and which would give an unpleasant feeling.

In comparison with the oily forms (ointment), the "gel" form of meclophenamic acid according to the invention is more compatible with the skin injuries and the exudates and, therefore, may be smeared more easily onto the injured part; it allows perspiration, which does not take place when using the oilbased topical form. In comparison with the pharmaceutical forms based on emulsions of oil in water or water in oil (cream or paste), the pharmaceutical "gel" form of the meclophenamic acid in a hydro-alcoholic medium guarantees a better acceptability (compliance) by the patient since, after the application, the treated zone results fully dry and with no greasy or sticky residual substances, contrary to what happens by utilizing the known forms containing oils or glycols.

The invention also refers to a composition using the above-mentioned pharmaceutical form and to the use of said form and composition for the production of a medicament for topical use, as defined in the appended claims.

Three examples of formulations are indicated below, reference being made to 100 g of "gel":

### Example I

N-(2.6-dichloro-m-tolyl)-anthranilic acid g 2.5
carboxypolymethylene at 2-3 . $10^6$ m.w. " 2.25
ethyl alcohol ml 20
esters of p-hydroxybenzoic acid g 0.1
lavender and neroli essence " 0.15
triethanolamine q.s. at 5.5–6.5 pH
water q.s. at g 100

### Example II

N-(2.6-dichloro-m-tolyl)-anthranilic acid g 5
carboxypolymethylene at 2-3 . $10^6$ m.w. " 2.25
ethyl alcohol ml 20
esters of p-hydroxybenzoic acid g 0.1
lavender and neroli essence " 0.15
triethanolamine q.s. at 5.5–6.5 pH
water q.s. at g 100

### Example III

N-(2,6-dichloro-m-tolyl)-anthranilic acid g 10
carboxypolymethylene at 2-3 . $10^6$ m.w. " 2.25
ethyl alcohol ml 20
esters of the p-hydroxybenzoic acid g 0.1
lavender and neroli essence " 0.15
triethanolamine q.s. at 5.5–6.5 pH
water q.s. g 100

In relation to the clinical experimentation, the "gels" based on meclophenamic acid of the cited examples have shown excellent local and general tolerability as well as therapeutic activity.

## Claims for the Contracting States BE, CH, DE, FR, GB, LI, LU, NL, SE

1. Pharmaceutical form containing N-(2.6-dichloro-m-tolyl)-anthranilic acid (meclophenamic acid) as an active compound for topical use, made up of a preparation of the product in "gel" form, characterized in that the active compound is suspended in a hydro-alcoholic medium not containing a glycol.

2. The use of pharmaceutical "gel" forms according to claim 1, for the preparation of a medicament for topical use.

3. Composition for pharmaceutical use according to claim 1, characterized by the following formulation:

N-(2,6-dichloro-m-tolyl)-anthranilic acid g 2.5 to 10
carboxypolymethylene at 2–3 x $10^6$ m.w. approx. g 2.25
ethyl alcohol " ml 20
esters of p-hydroxybenzoic acid " g 0.1

lavender and neroli essence " g 0.15
triethanolamine q.s. at 5.5–6.5 pH
water q.s. g 100

4. The use of the pharmaceutical composition of claim 3 for the preparation of an analgesic and anti-inflammatory medicament for topical use.

**Claims for the Contracting Sates AT, GR, ES**

1. Method for the preparation of a pharmaceutical form containing N-(2.6-dichloro-m-tolyl)-anthranilic acid (meclophenamic acid) as an active compound for topical use, made up of a preparation of the product in "gel" form, characterized in that the active compound is suspended in a hydro-alcoholic medium hot containing a glycol.

2. Method for the preparation of a medicament according to claim 1, characterized in that a pharmaceutical "gel" form of said N-(2,6-dichloro-m-tolyl)-anthranilic acid is suspended in a hydro-alcoholic medium.

3. Method for the preparation of a composition for pharmaceutical use in the topical form of "gel" according to claim 1, characterized by adding:

N-(2.6-dichloro-m-tolyl)-anthranilic acid 2.5 to 10 to
carboxypolymethylene at 2–3 . $10^6$ m.w.approx. g 2.25
ethyl alcohol " ml 20
esters of p-hydroxybenzoic acid " g 0.1
lavender and neroli essence " g 0.15
triethanolamine q.s. at 5.5–6.5 pH
water q.s. g 100

4. The use of the pharmaceutical composition of claim 3 for the preparation of an analgesic and anti-inflammatory medicament for topical use.

**Patentansprüche für die Vertragsstaaten: BE, CH, DE, FR, GB, LI, LU, NL, SE**

1. Arzneimittelform, die N-(2,6-Dichlor-m-tolyl)Anthranilsäure (Mechlophenaminsäure) als aktive Verbindung für lokale Anwendungen enthält und als Zubereitung des Produktes in Gelform ausgebildet ist, dadurch gekennzeichnet, daß die aktive Verbindung in einem wässrig-alkoholischen Medium suspendiert ist, welches kein Glykol enthält.

2. Anwendung von Gel-Arzneiformen nach Anspruch 1 für die Zubereitung eines Heilmittels für lokale Anwendungen.

3. Zusammensetzung nach Anspruch 1 für die Verwendung als Arzneimittel, gekennzeichnet durch die folgende Formulierung:

N-(2,6-Dichlor-m-tolyl)Anthranilsäure 2,5–10 g
Carboxypolymethylen bei 2–3 x $10^6$ m. w. etwa 2,25 g
Äthylakohol " 20 ml
Ester der p-Hydroxybenzoesäure " 0,1 g
Lavendel - und Neroliessenz " 0,15 g
Triäthanolamin q. s. ad 5,5–6,5 pH
Wasser q. s. 100 g

4. Verwendung der Arzneimittelzusammensetzung nach Anspruch 3 für die Zubereitung eines schmerzlindernden und entzündungshemmenden Heilmittels für lokale Anwendungen.

**Patentansprüche für die Vertragsstaaten AT, ES, GR**

1. Verfahren zur Herstellung einer Arzneimittelform, die N-(2,6-Dichlor-m-tolyl)Anthranilsäure (Mechlophenaminsäure) als aktive Verbindung für lokale Anwendungen enthält und als Zubereitung des Produktes in Gelform ausgebildet ist, dadurch gekennzeichnet, daß die aktive Verbindung in einem wässrig-alkoholischen Medium suspendiert wird, welches kein Glykol enthält.

2. Verfahren zur Herstellung eines Arzneimittels nach Anspruch 1, dadurch gekennzeichnet, daß eine pharmazeutische Gelform der N-(2,6-Dichlor-m-tolyl)Anthranilsäure in einem wässrig-alkoholischen Medium suspendiert wird.

3. Verfahren für die Zubereitung einer Zusammensetzung für die Verwendung als Arzneimittel in der lokal anwendbaren Form eines Gels nach Anspruch 1, dadurch gekennzeichnet, daß

N-(2,6-Dichlor-m-tolyl)Anthlanilsäure 2,5–10 g
zu Carboxypolymethylen bei 2–3 x $10^6$ m. w. etwa 2,25 g
Äthylakohol " 20 ml Ester der p-Hydroxybenzoesäure " 0,1 g
Lavendel - und Neroliessenz " 0,15 g
Triäthanolamin q. s. ad 5,5–6,5 pH
und
Wasser
q. s. 100 g
gegeben wird.

4. Verwendung der Arzneimittelzusammensetzung nach Anspruch 3 für die Zubereitung eines schmerzlindernden und entzündungshemmenden Heilmittels für lokale Anwendungen.

**Revendications pour les Etats contractants BE, CH, DE, FR, GB, LI, LU, NL, SE**

1. Forme pharmaceutique contenant de l'acide N-(2.6-dichloro-m-tolyl)-anthranilique, (acide méclophénamique) comme composé actif pour usage local, constituée d'une préparation du produit sous forme de "gel", caractérisée en ce que le composé actif est mis en suspension dans un milieu hydro-alcoolique ne contenant pas de glycol.

2. Utilisation de la forme pharmaceutique "gel" selon la revendication 1 pour la préparation d'un médicament pour usage local.

3. Composition selon la revendication 1, caractérisée par la formule suivante:

Acide N-(2.6-dichloro-m-totyl)-anthranilique 2,5 à 10 g
carboxypolyméthylène, MM 2–3 . $10^6$ env. 2,25 g
alcool éthylique 20 ml
esters de l'acide p-hydroxybenzoique 0,1 g
essence de lavande et de néroli 0,15 g
triéthanolamine q.s. pour pH 5,5–6,5
eau q.s. pour faire 100 g

4. Utilisation de la composition pharmaceutique de la revendication 3 pour la préparation d'un médicament analgésique et anti-inflammatoire pour l'usage local.

**Revendications pour les Etats contractants AT, GR, ES**

1. Méthode pour la préparation d'une forme pharmaceutique contenant de l'acide N-(2.6-dichloro-m-tolyl)-anthranilique (acide méclophénamique), comme composé actif pour usage local, constituée d'une préparation du produit sous forme de "gel", caractérisé en ce que le composé actif est mis en suspension dans un milieu hydro-alcoolique ne contenant pas de glycol.

2. Méthode pour la préparation d'un médicament selon la révendication 1, caracterisé en ce que une forme pharmaceutique "gel" de cet acide N-(2.6-dichloro-m-tolyl)-anthranilique est suspendue en milieu hydro-alcoolique.

3. Méthode pour la préparation d'une composition pour utilisation pharmaceutique en forme de "gel" pour l'usage local, selon la révendication 1, caractérisé par l'adjonction de:

Acide N-(2.6-dichloro-m-to]yl)-anthranilique 2,5 à 10 g

carboxypolyméthylène, MM 2–3 . 10[6] env. 2,25 g alcool éthylique " 20 ml

esters de l'acide p-hydroxybenzoique " 0,1 g

essence de lavande et de néroli " 0,15 g

triéthanolamine q.s. pour pH 5,5–6,5

eau q.s. pour faire 100 g

4. Utilisation de la composition pharmaceutique de la révendication 3 pour la préparation d'un médicament analgésique et anti-inflammatoire pour l'usage local.